# EUROPEAN PATENT SPECIFICATION

(11) **EP 2 538 922 B1**
(45) Date of publication and mention of the grant of the patent: **06.04.2016**
(21) Application number: 11705573.1
(22) Date of filing: 25.02.2011
(51) Int. Cl.: A61K 9/14, A61K 31/167, A61K 31/58

(54) **FORMULATION OF COMPOSITIONS FOR THE TREATMENT OF INFLAMMATORY CONDITIONS**
FORMULIERUNG VON ZUSAMMENSETZUNGEN ZUR BEHANDLUNG VON ENTZÜNDUNGSERKRANKUNGEN
FORMULATION DE COMPOSITIONS POUR LE TRAITEMENT D'ÉTATS INFLAMMATOIRES

(30) Priority: 02.03.2010 GB 201003462; 25.02.2010 GB 201003176
(43) Date of publication of application: 02.01.2013
(73) Proprietor: Andi-Ventis Limited, Limassol CY-3604 (CY)
(72) Inventor: RIPAMONTI, Dr Arnaldo, Limassol CY-3604 (CY)
(74) Representative: Gill Jennings & Every LLP
(86) International application number: PCT/EP2011/052847
(87) International publication number: WO 2011/104360

(56) References cited:
- WO-A1-01/89491
- WO-A1-99/64014
- WO-A1-2006/086270
- WO-A2-2004/017918

## Description

### Field of the Invention

The present invention relates to a process for the formulation of pharmaceutical compositions comprising a β₂-adrenergic agonist and a glucocorticosteroid.

### Background of the Invention

Pharmaceutical compositions comprising selective β₂-adrenergic agonists and glucocorticosteroids are used in the treatment of inflammatory conditions or disorders, in particular, respiratory diseases such as asthma, chronic obstructive pulmonary disease (COPD) and rhinitis. These compositions are generally formulated as stable powders, suitable for administration via oral or nasal inhalation.

Compositions for the treatment of respiratory disorders commonly comprise the compounds formoterol and budesonide, either in combination or as single formulations for separate, sequential administration.

Formoterol ((*RS*,*SR*)-*N*-[2-hydroxy-5-[1-hydroxy-2-[1-(4-methoxyphenyl)propan-2-ylamino]ethyl] phenyl]formamide) is a highly potent and selective β₂-adrenergic agonist with a long duration of action when inhaled. Compared with other β₂-adrenergic compounds it has a unique chemical structure with a formamido group substituted on the benzene ring. It also has two asymmetric carbon atoms in the molecule, making four stereoisomers possible. Inhaled formoterol works like other β₂-agonists, causing bronchodilation by relaxing the smooth muscle in the airway so as to treat the exacerbation of asthma. The long duration of formoterol action occurs because the formoterol molecules initially diffuse into the plasma membrane of the lung cells, and then are slowly released back outside, where they come into contact with β₂ adrenergic receptors. Formoterol has been demonstrated to have a faster onset of action than other β₂-adrenergic compounds as a result of lower lipophilicity, and has also been demonstrated to be more potent; a 12 µg dose of formoterol has been demonstrated to be equivalent to a 50 µg dose of salmeterol, which is a short-acting β₂-adrenergic compound.

Budesonide (16,17-(butylidenebis(oxy))-11,21-dihydroxy-,(11-β,16-a)-pregna-1,4-diene-3,20-dione) is a glucocorticosteroid which has a high first-pass metabolism and, unlike other corticosteroids, has little influence on the hypothalamic-pituitary-adrenal axis and has a lower incidence of systemic manifestations than similar compounds. As such, budesonide is a preferred glucocorticosteroid for use in compositions for the treatment of inflammatory conditions.

Pharmaceutical compositions intended for inhalation are generally formulated in association with carriers/diluents such as lactose, in order to facilitate dosing from the inhaler. These formulations generally consist of coarse particles of carrier together with fine particles of the drug(s), optionally together with small particles of the carrier/diluent. An alternative to such a formulation is to form agglomerates of the small particles of the drug(s) and the carrier/diluent.

Pharmaceutical compositions for the treatment of respiratory diseases comprising formoterol, or enantiomers thereof, as the selective β₂ -agonist component and budesonide as the glucocorticosteroid component are available commercially under the brand name Symbicort™. Compositions comprising formoterol as the only active ingredient are available under brand names including Foradil™, Oxeze™ and Performist™. Compositions comprising budesonide as the only active ingredient are available under the brand names Pulmicort™ (nasal inhalation) and Rhinocort™ (oral inhalation).

Current processes for formulating the above-described pharmaceutical compositions are described in prior art publications including EP1289506, EP1462100 and WO0189492, and comprise the following general steps:
1. mixing the first active ingredient with the carrier/diluent and micronising the mixture;
2. optionally adding further micronized carrier/diluent to the mixture;
3. adding and mixing the pre-micronized second active ingredient, which is optionally pre-mixed with micronized carrier/diluent; and
4. either subjecting the resulting mixture to agglomeration and spheronisation, or adding coarse carrier/diluent.
However, there are a number of disadvantages to this process, including the risk of undesirable agglomerates forming. Furthermore, the process is limited as it is very difficult to adjust the formulation of the composition once the process has reached an advanced stage.

Therefore, there is a need in the art for improved processes for formulating pharmaceutical compositions suitable for administration via oral or nasal inhalation.

Such improved processes provide high product quality and enable greater control and flexibility of the formulation process.

### Summary of the Invention

The present invention provides an improved process for the formulation of a pharmaceutical composition comprising a β₂-adrenergic agonist or a glucocorticosteroid comprising the steps of:
(i) mixing a coarse excipient and a microfine excipient and sieving the resulting mixture;
(ii) mixing a β₂-adrenergic agonist with a microfine excipient and sieving the resulting mixture;
(iii) adding the product of step (ii) to a portion of the mixture obtained in step (i);
(iv) blending and/or sieving the product obtained in step (iii);
   and separately
(v) mixing a glucocorticosteroid with a microfine excipient and sieving the resulting mixture;
(vi) adding the product of step (v) to a portion of the mixture obtained in step (i); and
(vii) blending and/or sieving the product obtained in step (vi),
wherein the glucocorticosteroid composition of step (vii) is combined with the β₂-adrenergic agonist composition of step (iv) and the resulting composition is sieved and blended.

The process of the present invention may also comprise the further step of diluting the product of step (iv) with the excipient mixture obtained according to step (i), and mixing and/or sieving to produce a final blend.

### Description of the Drawings

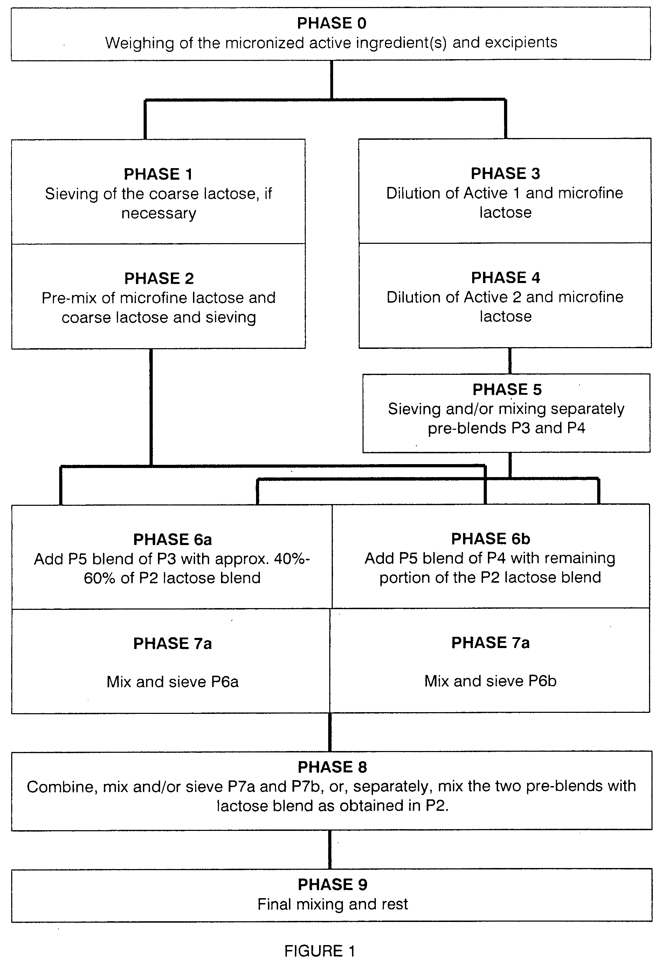

Figure 1 is a flow chart summarising the process of the invention.

### Detailed Description of the Invention

The present invention provides an improved process for the formulation of a pharmaceutical composition comprising a β₂-adrenergic agonist or a glucocorticosteroid. Such compositions, which are formulated as stable powders suitable for administration via oral or nasal inhalation, are useful in the treatment of inflammatory conditions or disorders, in particular respiratory diseases such as asthma, COPD and rhinitis.

The process for formulating the pharmaceutical composition comprises the steps of:
(i) mixing a coarse excipient and a microfine excipient and sieving the resulting mixture;
(ii) mixing a β₂-adrenergic agonist with the microfine excipient and sieving the resulting mixture;
(iii) adding the product of step (ii) to a portion of the mixture obtained in step (i);
(iv) blending and/or sieving the product obtained in step (iii);
   and separately
(v) mixing a glucocorticosteroid with a microfine excipient and sieving the resulting mixture;
(vi) adding the product of step (v) to a portion of the mixture obtained in step (i); and
(vii) blending and/or sieving the product obtained in step (vi),
wherein the glucocorticosteroid composition of step (vii) is combined with the β₂-adrenergic agonist composition of step (iv) and the resulting composition is sieved and blended.

In an embodiment, the process of the present invention comprises the additional step of diluting the product of step (iv) with the excipient mixture obtained according to step (i), and mixing and/or sieving to produce a final blend.

There are a number of advantages associated with the process of the invention. As illustrated in Figure 1, the two active ingredients (a β₂-adrenergic agonist and a glucocorticosteroid) are formulated entirely separately until the final stages of the process. This enables greater control and flexibility in the formulation process, as the two formulations can be adjusted separately prior to combination, or indeed not combined at all if single-active formulations are required.

As such, the process of the invention is a modular process; two separate formulations containing different active agents are prepared in parallel and it is only at the end of the process that it is necessary to decide whether to obtain two single-active formulations or a single formulation combining both active ingredients.

Unlike the process methodologies of the prior art in which the coarse excipient is added to the blend as a final step after the combination of the first and second actives, according to the present invention the coarse excipient is added during the initial stages of the process and is present at the stage that the two micronized actives are combined. This avoids the risk of agglomeration between the microfine actives caused by mixing two microfine actives in the absence of a carrier/support component.

A further advantage of formulating two products separately, rather than combining the active ingredients in the initial stages of the formulation process, is that different sieves, having different mesh numbers, can be used for each active agent. This results in blends having different properties and performance in terms of uniformity and respirable dose/fraction. This enables greater control of the behaviour of each of the actives, allowing different particle sizes of each of the actives, as desired according to the *in vivo* or *in vitro* application. This may be particularly important in the case of a combination of actives having different target receptors within the respiratory tract (such as glucocorticosteroids and β₂-adrenergic agonists). Such control is not possible if the actives are combined early in the formulation process (e.g. pre-mixed together and/or micronized together).

A rocking granulator is preferably used to simultaneously sieve and mix the components. The rocking movement of the head of the granulator forces the bulk material through a sieving net, thereby enabling greater control over the particle dimensions and the time taken to achieve the required product uniformity.

Finally, the process of the invention is advantageous as it is suitable for large-scale commercial batch production.

The term 'β₂-adrenergic agonist' is defined herein as any member of the class of compounds acting on the β₂-adrenergic receptor, thereby causing smooth muscle relaxation, resulting in dilation of bronchial passages, vasodilation in muscle and liver, relaxation of uterine muscle and release of insulin. Compounds within this class can be divided into two groups: short-acting and long-acting agonists. Short-acting β₂-agonists include salbutamol, levosalbutamol and terbutaline. Long-acting β₂-agonists include salmeterol, formoterol, bambuterol and clenbuterol. According to the present invention, long-acting agonists are preferred, most preferably formoterol, which can be in the form of a mixture of enantiomers or a single active enantiomer.

The term 'glucocorticosteroid' is defined herein as any member of the class of steroid hormones that bind to the glucocorticoid receptor, resulting in up-regulation of expression of anti-inflammatory proteins in the nucleus and down-regulation of expression of pro-inflammatory proteins in the cytosol. As such, this class of compounds is useful in the treatment of respiratory disorders resulting from an inflammatory response. Glucocorticosteroids suitable for use according to the present invention include fluticasone propionate, mometasone furoate, ciclesonide, budesonide, and epimers, esters, salts and solvates thereof. According to the present invention the preferred glucocorticosteroid is budesonide.

The composition formulated according to the process of the present invention comprises excipients which function as diluents/carriers. The excipient(s) may be any pharmaceutically-inactive powder compounds having suitable particle size and flow properties, including carbohydrates such as sucrose or lactose. A microfine excipient is defined as having a particle diameter size of less than 10µm, preferably 2-5µm. A coarse excipient is defined as having a particle diameter size of 150-400µm.

According to the present invention, it is preferred if the excipients are microfine lactose monohydrate and coarse lactose monohydrate. Lactose monohydrate is a naturally-occurring disaccharide, which consists of one glucose moiety and one galactose moiety and is obtained from milk. Microfine lactose monohydrate functions as a diluent. Coarse lactose monohydrate functions as a support/carrier, and is present in the composition throughout the formulation process in order to prevent the micronized particulate components from agglomerating.

Pharmaceutical compositions formulated according to the process of the invention may be administered via any suitable route, however the preferred administration routes are oral and nasal inhalation, which enable pulmonary delivery of the therapeutic agents(s).

An embodiment of the process of the present invention is summarised in Figure 1.

According to step (i) of the present invention, the coarse excipient, preferably coarse lactose, is mixed with the microfine excipient, preferably microfine lactose, and the resulting mixture is sieved. In a preferred embodiment, coarse excipient having a MMAD of 200-250µm is added to a quantity of microfine excipient, having a MMAD of 2-5µm, corresponding to about 1-10% of the final mass, preferably 2-6%. If the coarse excipient has been sieved in order to achieve the required particle size (Phase 1 of Figure 1), it should preferably be left to rest overnight before mixing with the microfine excipient, in order to allow for electro-static discharge. The mixture of coarse and microfine excipients is roughly mixed and sieved through a mesh having a pore diameter of 500-900µm, preferably a pore diameter of 600-700µm, and then mixed for 10-40 minutes, preferably 10-30 minutes and most preferably 20-30 minutes, at 6rpm with a rotating mixer (Phase 2 of Figure 1). It is preferable to use a rocking granulator when sieving the mixture. The obtained blend is left to rest overnight in order to allow for electro-static discharge.

According to step (ii) of the process of the invention, an active ingredient, either the β₂-adrenergic agonist, preferably formoterol, or the glucocorticosteroid, preferably budesonide, is added to a portion of microfine excipient in a gradual, multi-step manner (Phase 3 of Figure 1). Preferably, the active ingredients are pre-micronized prior to use in the process of the invention, such that at least 80%, preferably 85%, more preferably 90% and most preferably 95% of the active has a MMAD of less than 5µm. Such small particle sizes are necessary in order to ensure that the active agents reach the lungs following inhalation.

If the active ingredient is the β₂-adrenergic agonist, it is preferably added to the microfine excipient in a ratio of 1:20 - 1:1, more preferably 1:10. If the active ingredient is the glucocorticosteroid, it is preferably added to the microfine excipient in a ratio of 3:1 - 1:3, more preferably 1:1. The resulting blend is then sieved using a mesh having a pore diameter of 400-700µm, preferably a pore diameter of 500-600µm, and left to rest overnight (Phase 5 of Figure 1; 5a and 5b correspond to the two different actives). This step can optionally be carried out separately with the other active agent (Phase 4 of Figure 1), resulting in two separate blends (5a and 5b), each comprising of a different active agent (i.e. one comprising a β₂-adrenergic agonist and the other comprising a glucocorticosteroid) and microfine excipient.

The one or both blends obtained in step (ii) are then added, separately, to a portion of the blend of coarse and microfine excipients obtained in step (i) (Phases 6a and/or 6b of Figure 1). It is preferred if 25%-75% of the total active agent blends is added to between 25%-75%, preferably 40%-60%, and most preferably 50%, of the coarse/microfine lactose blend. The resulting mixture(s) is/are blended roughly in a cubic mixer and sieved using a mesh having a pore diameter of 400-1000 µm, preferably a pore diameter of 500-800µm, more preferably a pore diameter of 600-700µm, to produce a semi-finished blend (Phases 7a and 7b of Figure 1). It is preferable to use a rocking granulator when sieving the mixture.

The next step is the preparation of the final blend (Phase 8 of Figure 1). At this stage it is possible to obtain any of three formulations: a combination formulation comprising both active agents; a formulation comprising the β₂-adrenergic agonist as the only active agent; and/or a formulation comprising the glucocorticosteroid as the only active agent.

In order to obtain a combination formulation, the two semi-finished blends are combined and diluted with a coarse/microfine excipient blend equivalent to that obtained in step (i). The resulting blend is mixed for 1-10minutes, preferably 1-5 minutes, and most preferably 1-3 minutes, and/or directly sieved through a mesh having a pore diameter of 400-1000µm, preferably a pore diameter of 500-800µm and more preferably a pore diameter of 600-700µm. Again, it is preferable to use a rocking granulator when sieving the mixture. The final blend is mixed again for 1-5 minutes, preferably 1-2 minutes, at 6-10 rpm with a rotating mixer and left to rest overnight for electrostatic discharge.

In order to obtain separate formulations, the one or two semi-finished blend(s) is/are (separately) diluted with a coarse/microfine excipient blend equivalent to that obtained in step (i). The resulting blend(s) is/are mixed together for 1-5 minutes, preferably 1-2 minutes, and/or directly sieved through a mesh having a pore diameter of 500-900µm, preferably a pore diameter of 600-700µm, with a rocking granulator. The final blend(s) is/are mixed again for 1-5 minutes, preferably 1-2 minutes, at 6-10 rpm with a rotating mixer and left to rest overnight for electrostatic discharge (Phase 9 of Figure 1).

Table 1 details examples of nine different budesonide/formoterol combination formulations.

**Table 1**

| **Batch** | **1** | **2** | **3** | **4** | **5** | **6** | **7** | **8** | **9** |
|---|---|---|---|---|---|---|---|---|---|
| Theoretical Batch size (g) | 2000 | 2000 | 1000 | 2000 | 2000 | 2000 | 2000 | 2000 | 2000 |
| **Formula** | **80/4**.**5** | **(80 4.5)** | **(160/4**.**5)** | **(160/4.5)** | **(160/4.5)** | **(160/4**.**5)** | **(160/4.5)** | **(80/4.5)** | **(80/4.5)** |
| Budesonide | 90 | 90 | 180 | 192 | 200 | 206.43 | 206.44 | 103.22 | 101.59 |
| Formoterol fumarate dihydrate | 5.4 | 5.4 | 5.4 | 5.4 | 5.63 | 5.81 | 5.81 | 5.81 | 5.71 |
| Ratio (Budesonide: formoterol) | 16.67 | 16.67 | 33.33 | 35.56 | 35.52 | 35.55 | 35.56 | 17.78 | 17.78 |
| Microfine lactose monohydrate | 315 | 315 | 136.8 | 380 | 310 | 300 | 340 | 340 | 340 |
| Coarse lactose monohydrate | 3189.6 | 3189.6 | 3277.8 | 3022.6 | 3484.4 | 3487.8 | 3447.8 | 3551.0 | 3552.7 |
| | | | | | | | | | |
| Budesonide % | 2.50 | 2.50 | 5.00 | 5.333 | 5.000 | 5.161 | 5.161 | 2.580 | 2.540 |
| Formoterol % | 0.15 | 0.15 | 0.15 | 0.15 | 0.141 | 0.145 | 0.145 | 0.145 | 0.143 |
| Microfine lactose % | 8.75 | 8.75 | 3.80 | 10.556 | 7.750 | 7.500 | 8.500 | 8.500 | 8.500 |
| Coarse lactose % | 88.60 | 88.60 | 91.05 | 83.961 | 87.109 | 87.194 | 86.194 | 88.774 | 88.817 |

As described above, the process of the invention enables greater control and flexibility in the formulation of the resulting pharmaceutical composition(s), as two separate formulations can be adjusted prior to combination, or indeed not combined at all if single-active formulations are required.

As a result of adding coarse excipient to the formulations during the initial stages of the process, agglomeration between the microfine actives is reduced and the required product uniformity is achieved in the resulting pharmaceutical compositions in a controlled manner.

Furthermore, the compositions obtained by the process of the invention, such as those disclosed in Table 1, are suitable for up-scaling to quantities required for commercial batch production.

## Claims

1. A process for the production of a pharmaceutical composition comprising a β₂-adrenergic agonist and a glucocorticosteroid, comprising the steps of:
(i) mixing a coarse excipient and a microfine excipient and sieving the resulting mixture;
(ii) mixing a β₂-adrenergic agonist with a microfine excipient and sieving the resulting mixture;
(iii) adding the product of step (ii) to a portion of the mixture obtained in step (i);
(iv) blending and/or sieving the product obtained in step (iii); and separately
(v) mixing a glucocorticosteroid with a microfine excipient and sieving the resulting mixture;
(vi) adding the product of step (v) to a portion of the mixture obtained in step (i); and
(vii) blending and/or sieving the product obtained in step (vi),
wherein the glucocorticosteroid composition of step (vii) is combined with the β₂-adrenergic agonist composition of step (iv) and the resulting composition is sieved and blended.

2. A process according to claim 1, further comprising the step of diluting the products of steps (iv) and (vii) with the excipient obtained according to step (i) and mixing and/or sieving to produce a final blend.

3. A process according to claim 1 or claim 2, wherein the resulting composition is sieved using sieves having a pore diameter between 400µm-1000µm, preferably 500µm-800µm.

4. A process according to any preceding claim, wherein steps (ii) and/or (v) are carried out using sieves having a pore diameter between 400µm-700µm, preferably 500µm - 600µm.

5. A process according to any preceding claim, wherein steps (iv) and/or (vii) are carried out using sieves having a pore diameter between 400µm-1000µm, preferably 500µm -800µm.

6. A process according to any preceding claim, wherein step (ii) is carried out by adding a β₂-adrenergic agonist to the microfine excipient in a ratio of between 1:20 to 1:1, preferably 1:10.

7. A process according to any preceding claim, wherein step (v) is carried out by adding a glucocorticosteroid to the microfine excipient in a ratio of between 3:1 to 1:3, preferably 1:1.

8. A process according to any preceding claim, wherein steps (iii) and (vi) are carried out by adding 25%-75%, preferably 40%-60%, of the product of step (i) to 25%-75% of the product of steps (ii) and (v).

9. A process according to any preceding claim, wherein the β₂-adrenergic agonist is formoterol, and/or the glucocorticosteroid is budesonide.

10. A process according to any preceding claim, wherein the coarse and microfine excipients are carbohydrates, preferably wherein the coarse excipient is coarse lactose monohydrate, and/or wherein the microfine excipient is microfine lactose monohydrate.

11. A process according to any preceding claim, wherein the β₂-adrenergic agonist and the glucocorticosteroid are pre-micronized.

12. A process according to any preceding claim, wherein a rocking granulator is used to assist in sieving the components.

## Patentansprüche

1. Verfahren für die Herstellung einer pharmazeutischen Zusammensetzung, die einen β₂-adrenergen Agonisten und ein Glucocorticosteroid umfasst, das die folgenden Schritte umfasst:
(i) Mischen eines groben Hilfsstoffs und eines mikrofeinen Hilfsstoffs und Sieben der entstehenden Mischung;
(ii) Mischen eines β₂-adrenergen Agonisten mit einem mikro feinen Hilfsstoff und Sieben der entstehenden Mischung;
(iii) Zugeben des Produkts von Schritt (ii) zu einem Teil der Mischung, die im Schritt (i) erhalten wurde;
(iv) Vermischen und/oder Sieben des Produkts, das im Schritt (iii) erhalten wurde; und separat
(v) Mischen eines Glucocorticosteroids mit einem mikrofeinen Hilfsstoff und Sieben der entstehenden Mischung;
(vi) Zugeben des Produkts von Schritt (v) zu einem Teil der Mischung, die im Schritt (i) erhalten wurde; und
(vii) Vermischen und/oder Sieben des Produkts, das im Schritt (vi) erhalten wurde,
wobei die Zusammensetzung des Glucocorticosteroids von Schritt (vii) mit der Zusammensetzung des β₂-adrenergen Agonisten von Schritt (iv) vereinigt wird und die entstehende Zusammensetzung gesiebt und vermischt wird.

2. Verfahren nach Anspruch 1, das weiter den Schritt des Verdünnens der Produkte der Schritte (iv) und (vii) mit dem Hilfsstoff, der entsprechend Schritt (i) erhalten wurde, und des Mischens und/oder Siebens umfasst, um ein Endgemisch herzustellen.

3. Verfahren nach Anspruch 1 oder Anspruch 2, wobei die entstehende Zusammensetzung unter Verwendung von Sieben, die einen Porendurchmesser zwischen 400 µm-1000 µm, bevorzugt 500 µm-800 µm aufweisen, gesiebt wird.

4. Verfahren nach einem vorstehenden Anspruch, wobei die Schritte (ii) und/oder (v) unter Verwendung von Sieben, die einen Porendurchmesser zwischen 400 µm-700 µm, bevorzugt 500 µm-600 µm aufweisen, durchgeführt werden.

5. Verfahren nach einem vorstehenden Anspruch, wobei die Schritte (iv) und/oder (vii) unter Verwendung von Sieben, die einen Porendurchmesser zwischen 400 µm-1000 µm, bevorzugt 500 µm-800 µm aufweisen, durchgeführt werden.

6. Verfahren nach einem vorstehenden Anspruch, wobei der Schritt (ii) durch Zugeben eines β₂-adrenergen Agonisten zu dem mikrofeinen Hilfsstoff in einem Verhältnis von zwischen 1 : 20 bis 1 : 1, bevorzugt 1 : 10 durchgeführt wird.

7. Verfahren nach einem vorstehenden Anspruch, wobei der Schritt (v) durch Zugeben eines Glucocorticosteroids zu dem mikrofeinen Hilfsstoff in einem Verhältnis von zwischen 3 : 1 bis 1 : 3, bevorzugt 1 : 1 durchgeführt wird.

8. Verfahren nach einem vorstehenden Anspruch, wobei die Schritte (iii) und (vi) durch Zugeben von 25 %-75 %, bevorzugt 40 %-60 % des Produkts von Schritt (i) zu 25 %-75 % des Produkts der Schritte (ii) und (v) durchgeführt wird.

9. Verfahren nach einem vorstehenden Anspruch, wobei der β₂-adrenerge Agonist Formoterol ist und/oder das Glucocorticosteroid Budesonid ist.

10. Verfahren nach einem vorstehenden Anspruch, wobei der grobe und der mikrofeine Hilfsstoff Kohlenhydrate sind, bevorzugt wobei der grobe Hilfsstoff grobes Lactose-Monohydrat ist und/oder wobei der mikrofeine Hilfsstoff mikro feines Lactose-Monohydrat ist.

11. Verfahren nach einem vorstehenden Anspruch, wobei der β₂-adrenerge Agonist und das Glucocorticosteroid zuvor mikronisiert werden.

12. Verfahren nach einem vorstehenden Anspruch, wobei ein schwingender Granulator verwendet wird, um das Sieben der Komponenten zu unterstützen.

## Revendications

1. Procédé pour la production d'une composition pharmaceutique comprenant un agoniste β₂-adrénergique et un glucocorticostéroïde, comprenant les étapes consistant à :
(i) mélanger un excipient grossier et un excipient ultrafin et passer le mélange résultant à travers un tamis:
(ii) mélanger un agoniste β₂-adrénergique avec un excipient ultrafin et passer le mélange résultant à travers un tamis ;
(iii) ajouter le produit de l'étape (ii) à une partie du mélange obtenu à l'étape (i) ;
(iv) mélanger et/ou passer le produit obtenu à l'étape (iii) à travers un tamis ; et séparément
(v) mélanger un glucocorticostéroïde avec un excipient ultrafin et passer le mélange résultant à travers un tamis ;
(vi) ajouter le produit de l'étape (v) à une partie du mélange obtenu à l'étape (i) ; et
(vii) mélanger et/ou passer le produit obtenu à l'étape (vi) à travers un tamis,
dans lequel la composition de glucocorticostéroïde de l'étape (vii) est combinée à la composition de l'agoniste β₂-adrénergiquede l'étape (iv) et la composition résultante est passée à travers un tamis et mélangée.

2. Procédé selon la revendication 1, comprenant en outre l'étape de dilution des produits des étapes (iv) et (vii) avec l'excipient obtenu selon l'étape (i) et mélanger et/ou passer à travers un tamis pour produire un mélange final.

3. Procédé selon la revendication 1 ou la revendication 2, dans lequel la composition résultante est passée à travers un tamis en utilisant des tamis ayant un diamètre de pores compris entre 400 µm et 1000 µm, de préférence 500 µm et 800 µm.

4. Procédé selon l'une quelconque des revendications précédentes, dans lequel les étapes (ii) et/ou (v) sont effectuées en utilisant des tamis ayant un diamètre de pores compris entre 400 µm et 700 µm, de préférence 500 µm et 600 µm.

5. Procédé selon l'une quelconque des revendications précédentes, dans lequel les étapes (iv) et/ou (vii) sont effectuées en utilisant des tamis ayant un diamètre de pores compris entre 400 µm et 1000 µm, de préférence 500 µm et 800 µm.

6. Procédé selon l'une quelconque des revendications précédentes, dans lequel l'étape (ii) est effectuée en ajoutant un agoniste β₂-adrénergique à l'excipient ultrafin dans un rapport compris entre 1:20 et 1:1, de préférence 1:10.

7. Procédé selon l'une quelconque des revendications précédentes, dans lequel l'étape (v) est effectuée en ajoutant un glucocorticostéroïde à l'excipient ultrafin dans un rapport compris entre 3 :1 et 1 :3, de préférence 1 : 1.

8. Procédé selon l'une quelconque des revendications précédentes, dans lequel les étapes (iii) et (vi) sont effectuées en ajoutant 25 % à 75 %, de préférence 40 % à 60 % du produit de l'étape (i) à 25 % à 75 % du produit des étapes (ii) et (v).

9. Procédé selon l'une quelconque des revendications précédentes, dans lequel l'agoniste β₂-adrénergique est du formotérol et/ou le glucocorticostéroïde est du budésonide.

10. Procédé selon l'une quelconque des revendications précédentes, dans lequel les excipients grossiers et ultrafins sont des hydrates de carbone, de préférence dans lequel l'excipient grossier est un monohydrate de lactose grossier et/ou dans lequel l'excipient ultrafin est un monohydrate de lactose ultrafin.

11. Procédé selon l'une quelconque des revendications précédentes, dans lequel l'agoniste β₂-adrénergique et le glucocorticostéroïde sont pré-micronisés.

12. Procédé selon l'une quelconque des revendications précédentes, dans lequel un granulateur oscillant est utilisé comme aide au tamisage des composants.
